# EUROPÄISCHE PATENTANMELDUNG

(11) **EP 0 633 002 A1**
(43) Veröffentlichungstag der Anmeldung: **11.01.1995**
(21) Anmeldenummer: 94107669.7
(22) Anmeldetag: 18.05.1994
(51) Int. Cl.: A61B 17/28

(54) **Chirurgische Zange**

(30) Priorität: 10.07.1993 DE 4323093
(71) Anmelder: Richard Wolf GmbH, D-75438 Knittlingen (DE)
(72) Erfinder: Falk, Ernst, D-75438 Knittlingen (DE)
(74) Vertreter: Wilcken, Thomas, Dipl.-Ing.

(57) **Zusammenfassung**

Die Zange hat eine Handhabe (1) und einen in diese einsetzbaren Zangeneinsatz, bestehend aus einem Schaft (3) mit einem an dessen distalem Ende angeordneten Zangenmaul (4). In dem Schaft (3) ist eine Stange (6) zum Öffnen und Schließen des Zangenmauls (4) geführt. Die Stange (6) ist mit dem Schaft (3) lösbar verriegelt und gegen Axialbewegung gesichert. Die Stange (6) und der Schaft (3), d.h. der Zangeneinsatz, sind mit der Handhabe (1) lösbar und drehbar verbunden, wobei die Stange (6) an ihrem proximalen Ende als Kupplungsteil (17, 18) ausgebildet ist, das lösbar mit der Handhabe (1) in Verbindung steht. Die beschriebene Zange ist ohne Zuhilfenahme von Werkzeugen, insbesondere zum Zwecke der wirksamen Reinigung und Desinfektion, leicht zerlegbar, so daß die Handhabe (1) auch mit unterschiedlichen Zangeneinsätzen bestückt werden kann.

## Beschreibung

Die Erfindung geht aus von einer chirurgischen Zange, insbesondere Zange zum Koagulieren von Gewebe mit HF-Strom, bei der das Zangenmaul durch zwei an einem Träger angeordnete Maulteile gebildet ist, von denen wenigstens eines relativ zum anderen und zum Träger mittels einer Stange zum Öffnen und Schließen des Zangenmauls verschwenkbar ist, bei der die durch einen Schaft verlaufende Stange bei Betätigung einer Handhabe axial im Schaft verstellbar ist.

Bei solchen Zangen (EP-A-0 484 671, EP-A-0 518 230) wird die Verdrehbarkeit des Zangenschaftes, gemeinsam mit der durch den Schaft verlaufenden Stange und mit den Maulteilen relativ zu dem feststehenden Teil der Handhabe, durch ein Betätigungselement erreicht, welches drehbar an der Handhabe angeordnet ist. Alle Teile dieser bekannten Zange sind werkseitig in fester Zuordnung zueinander montiert und bilden eine Funktionseinheit, die nur im ganzen gereinigt und desinfiziert werden kann.

Der Erfindung liegt die Aufgabe zugrunde, eine solche Zange dahingehend zu verbessern, daß sie auf einfache Weise demontiert und gründlich gereinigt, desinfiziert oder sterilisiert werden kann.

Diese Aufgabe wird erfindungsgemäß so gelöst, daß bei der eingangs erwähnten Zange der Träger und das distale Ende des Schaftes lösbar miteinander verriegelt und gegen Axialbewegung relativ zueinander gesichert sind, daß die Stange und der Schaft im Bereich der Handhabe lösbar und drehschlüssig verbunden sind und daß der proximale Endbereich der Stange als Kupplungsteil ausgebildet ist, das lösbar mit der Handhabe in Verbindung steht.

Vorzugsweise sind der Träger und der Schaft mittels einer bajonettartigen Verbindung verriegelt, wobei radiale Vorsprünge am Träger in Ausnehmungen des Schaftes greifen.

Die Demontage einer derart ausgebildeten Zange ist ohne Zuhilfenahme von Werkzeugen möglich und erlaubt eine individuelle Reinigungs- und Desinfektionsbehandlung der Zangenteile. Ein weiterer Vorteil der erfindungsgemäßen Zange liegt darin, daß die lösbare Ausbildung der Verbindung zwischen Stange und Schaft im Bereich der Handhabe lösbar und der proximale Endbereich der Stange als mit der Handhabe lösbar verbundenes Kupplungsteil auch die Anwendung unterschiedlicher Zangeneinsätze ermöglicht, mit denen die Handhabe wahlweise bestückbar ist.

Ein z.B. am Schaft vorgesehenes Federelement kann in eine Ausnehmung der Stange greifen und im Zusammenwirken mit dieser Ausnehmung die drehschlüssige Verbindung zwischen der Stange und dem Schaft bilden, wobei das Federelement zum Lösen dieser Verbindung durch Verdrehen der Stange bzw. des Maulteiles gegenüber dem Schaft selbsttätig außer Eingriff mit der Ausnehmung gelangt.

Bei einer monopolaren Zange mit einer durch die Maulteile gebildeten Elektrode und einem an der Handhabe angeordneten HF-Anschluß erfolgt die elektrische Verbindung zwischen Maulteil und HF-Anschluß über entsprechende elektrische Leiter sowie über eine den Schaft proximal aufnehmende Zylinderhülse und über den Schaft selbst oder über die am Maulteil angreifende Stange.

Weiter läßt sich eine stabile Festlegung des durch den Schaft einerseits und die Stange mit den an ihr befestigten Teilen andererseits gebildeten Zangeneinsatzes an der Handhabe mittels einer Gewindehülse erreichen, die mit einem Gewindestutzen am feststehenden Teil der Handhabe verschraubt ist.

Die Erfindung wird nachstehend anhand eines in der Zeichnung dargestellten Ausführungsbeispiels näher erläutert. Es zeigen:
- Figur 1: eine Seitenansicht der Zange,
- Figuren 2a und 2b: Seitenansichten des Zangenmauls mit unterschiedlich ausgebildeten Betätigungsstangen zum Öffnen und Schließen des Zangenmauls,
- Figuren 3a und 3b: Ansichten von Zangenschäften,
- Figur 4: eine Stirnansicht des distalen Endes des Schaftes,
- Figur 5: einen Schnitt durch die Handhabe nach Figur 1,
- Figur 6: die Handhabe nach Figur 5 in Entkuppelstellung und
- Figur 7: einen Teilschnitt durch die Handhabe in größerem Maßstab.

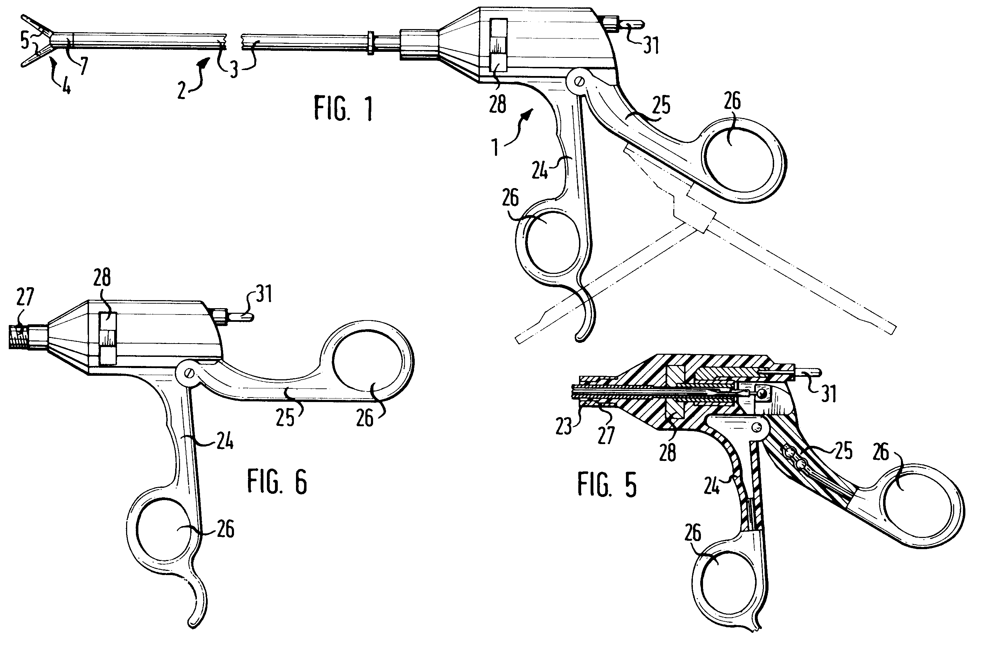

Gemäß Figur 1 hat die Zange eine Handhabe 1 und einen in diese einsetzbaren Zangeneinsatz 2, bestehend aus einem Schaft 3 mit einem an dessen distalem Ende angeordneten Zangenmaul 4 aus zwei Maulteilen 5 und einer in dem Schaft 3 geführten, nachfolgend kurz Stange genannten Zug- und Druckstange 6 zum Öffnen und Schließen des Zangenmauls 4.

Das Zangenmaul 4 ist in einem Träger 7 angeordnet, der durch eine gabelförmige Aufnahme 8 mit einer sich proximalseitig anschließenden Zylinderhülse 9 gebildet ist. In der Aufnahme 8 ist ein Gelenkstift 10 beidendig festgelegt, um den die beiden Maulteile 5 verschwenkbar gelagert sind. Die Maulteile 5 sind mit proximalseitigen Verlängerungen 11 versehen und mit dem distalen Ende der Stange 6 in bekannter Weise gelenkig verbunden. Die Zylinderhülse 9 weist an ihrem Übergang zu der Aufnahme 8 einen Bund 12 auf und ist mit zwei sich diametral gegenüberliegenden Vorsprüngen 13 ausgestattet, die mit Abstand zum Bund 12 angeordnet sind.

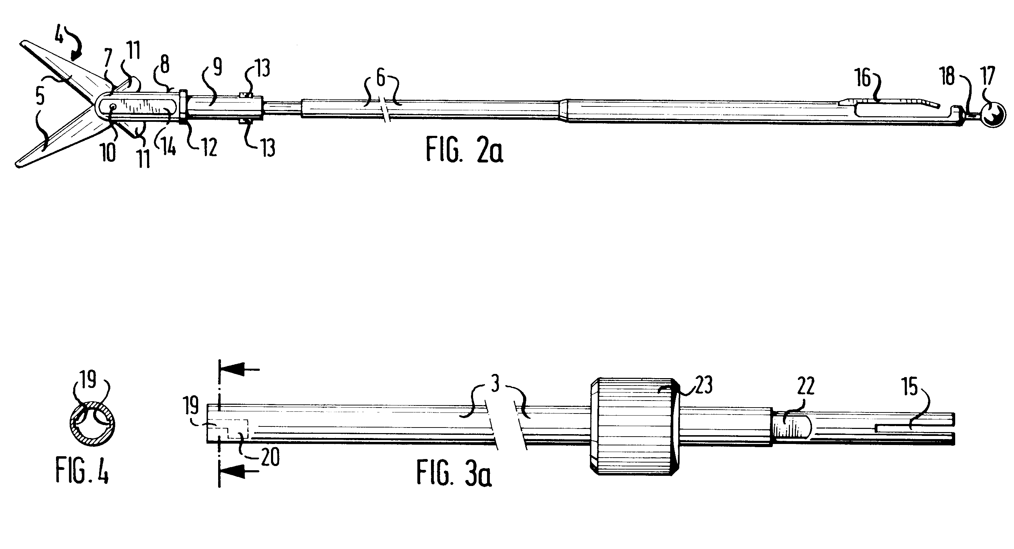

Die Stange 6 ist mit ihrem distalen Ende in einer zentralen Längsbohrung der Zylinderhülse 9 verschiebbar geführt und mündet in den zwischen den Schenkeln der gabelförmigen Aufnahme 8 befindlichen Zwischenraum 14. An seinem proximalen Endbereich ist der Schaft 3 mit einem Längsschlitz 15 versehen, der einen Freiraum für ein Federelement 16 bildet (Figuren 2a und 3a). Das proximale Ende der Stange 6 ist als Kugelelement 17 ausgebildet, welches an einem im Durchmesser reduzierten Stangenabschnitt 18 sitzt.

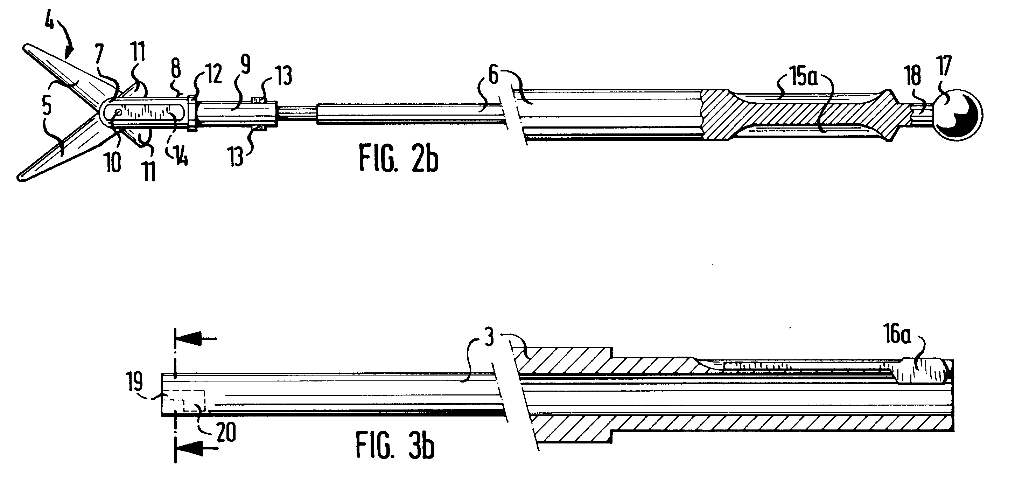

Die in den Figuren 2b und 3b dargestellten Baugruppen stimmen im Prinzip mit den in den Figuren 2a und 3a dargestellten überein und unterscheiden sich von diesen nur dadurch, daß das Federelement 16a am Schaft 3 und der Freiraum für das Federelement in Form von Ausnehmungen 15a an der Stange 6 vorgesehen ist.

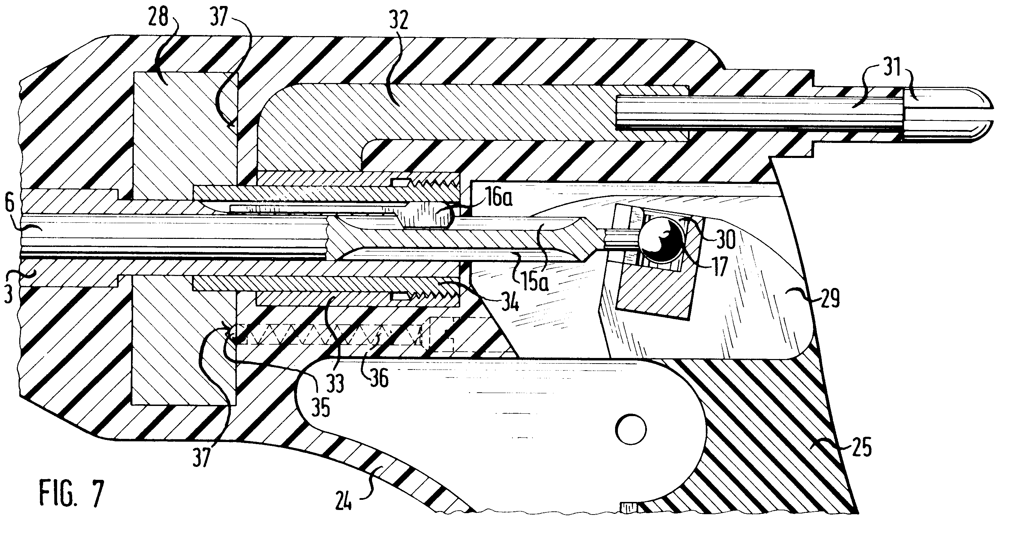

Der Schaft 3 ist rohrförmig und an seinem distalen Ende mit zwei an der Innenwand angeordneten, sich diametral gegenüberliegenden Längsnuten 19 (Figur 4) versehen, die in eine Ausnehmung 20 münden. Die Länge der Nuten 19 entspricht dabei dem Abstand der Vorsprünge 13 zum Bund 12, und die Länge der Ausnehmung 20 entspricht der Länge der Vorsprünge 13 plus einem gewissen Aufmaß. An seinem proximalen Ende ist der in Figur 3a gezeigte Schaft 3 mit dem Längsschlitz 15 und weiter distalwärts mit zwei sich diametral gegenüberliegenden Abflachungen 22 versehen. Schließlich ist der Schaft 3 mit einem nicht gezeigten Kragen ausgestattet, gegen dessen distalseitige Stirnfläche sich eine Gewindehülse 23 in Form einer Überwurf-Rändelmutter abstützt.

Die Handhabe 1 umfaßt gemäß Figuren 1 und 5 bis 7 einen feststehenden Teil 24 und einen schwenkbeweglichen, an diesem gelagerten Handgriff 25, die beide mit Grifflöchern 26 versehen sind. Der Teil 24 der Handhabe 1 weist eine Längsbohrung zur Aufnahme des proximalen Endteils des Schaftes 3 auf und ist an seinem distalwärts weisenden Ende mit einem von der Längsbohrung durchdrungenen Gewindestutzen 27 zur Aufnahme der Gewindehülse 23 versehen. An dem Teil 24 der Handhabe 1 befindet sich weiter ein Drehelement 28, das in einem Querschlitz des Teils 24 und zentral zu dessen Längsbohrung drehbar gelagert ist und einen zu der Abflachung 22 am Schaft 3 komplementär ausgerichteten Durchbruch hat.

Der bewegliche Handgriff 25 weist bei der Ausführung nach Figur 7 an seinem Schenkel 29 einen Kuppelmechanismus auf, bestehend aus einer nach oben offenen schlüssellochartigen Ausnehmung 30, in der das Kugelelement 17 der Stange 6 gehalten wird.

Am Teil 24 der Handhabe 1 ist ein HF-Anschluß 31 angebracht, der wahlweise an der Oberseite oder Rückseite der Handhabe vorgesehen sein kann und mit den Maulteilen 5 in elektrischer Verbindung steht.

Die vorstehend beschriebene Zange kann ohne Werkzeug zerlegt und montiert werden. Bei der Montage, beispielsweise nach einer Reinigung und Desinfektion, wird zweckmäßigerweise so vorgegangen, daß zunächst der Zangeneinsatz 2 zusammengebaut wird. Dazu wird die in Figur 2a bis 2b dargestellte Baugruppe, d.h. das Zangenmaul 4 mit der Stange 6 mit deren proximalen Ende voran in den in Figur 3a bzw. 3b gezeigten Schaft 3 von dessen Distalseite her eingeschoben, bis die Zylinderhülse 9 in den Schaft 3 eintritt. Die Zylinderhülse 9 bzw. die Stange 6 ist dabei vorab so auf den Schaft 3 ausgerichtet worden, daß die Vorsprünge 13 in die Längsnuten 19 gleiten können, bis der Bund 12 des Trägers 7 an dem distalen Ende des Schaftes 3 zur Anlage kommt.

Damit haben bei Verwendung der in den Figuren 2a und 3a gezeigten Baugruppen das Federelement 16 an dem proximalen Ende der Stange 6 den Bereich des Längsschlitzes 15 und die Vorsprünge 13 den Bereich der Ausnehmung 20 an dem distalen Ende des Schaftes 3 erreicht. Durch Relativverdrehung des Zangenmauls 4 mit der Stange 6 und des Schaftes 3 zueinander wird nun das Federelement 16 in den Längsschlitz 15 einschnappen, so daß eine drehschlüssige Verbindung dieser Teile erfolgt. Gleichzeitig wird so auch eine Axialverriegelung erreicht, da die Vorsprünge 13 außer Eingriff mit den Längsnuten 19 kommen und in der Ausnehmung 20 arretiert werden.

Bei Anwendung der Stange 6 und des Schaftes 3 gemäß den Figuren 2b und 3b wird in entsprechender Weise vorgegangen. Wenn also die Stange 6 vollständig in den Schaft 3 eingeführt ist, wird durch Verdrehung eines dieser Teile relativ zum anderen das radial nach innen vorgespannte Federelement 16a in eine der Ausnehmungen 15a schnappen und die Teile 3, 6 gegen weiteres Verdrehen sichern, während deren Sicherung gegen Axialbewegung wie vorher im Zusammenhang mit den Figuren 2a, 3a beschrieben erfolgt. Da sich bei diesem Ausführungsbeispiel die Feder 16a am Schaft 3 befindet, dessen proximalseitiges Ende im Bereich des starren Handhabenteiles 24 von einer zylindrischen Hülse 34 umgeben ist, wird vermieden, daß die Feder 16a ihre Position verändert. Da die Feder nicht radial nach außen ausgelenkt werden kann, ist eine Entnahme der Stange 6 aus dem Schaft 3 nicht möglich.

Der so gebildete Zangeneinsatz 2 kann nun mit dem proximalen Ende voran in die Längsbohrung im Teil 24 der Handhabe 1 eingeschoben werden. Vorher ist bei Ausführung der Handhabe 1 nach den Figuren 5 und 7 der Handgriff 25 in die Stellung entsprechend Figur 6 geschwenkt. Beim Einschieben des Zangeneinsatzes 2 wird das Drehelement 28 durchdrungen, und es kommen schließlich die Abflachungen 22 in dem Drehelement 28 zu liegen, um mit diesem eine drehschlüssige Verbindung einzugehen. Gleichzeitig wird der Handgriff 25 in die in Figur 5 und 7 gezeigte Stellung rückgeschwenkt und die Stange 6 durch Eingreifen des Kugelelementes 17 in die Schlüssellochausnehmung 30 mit dem Handgriff 25 gekuppelt. Durch Aufschrauben der Gewindehülse 23 auf den Gewindestutzen 27 wird dann der Zangeneinsatz 2 mit der Handhabe 1 fest verbunden.

Für eine Demontage der Zange wird die Verbindung zwischen Stange 6 und Handgriff 25 in umgekehrter Weise wie vorbeschrieben entkuppelt und nach Lösen der Gewindehülse 23 der Zangeneinsatz 2 aus der Handhabe 1 herausgezogen.

Der Schaft 3 und die Stange 6 werden durch Herausdrücken des Federelementes 16 aus dem Längsschlitz 15 entsperrt (Figur 2a und 3a), und dann wird die Stange relativ zum Schaft unter Zug verdreht, bis die Vorsprünge 13 mit den Längsnuten 19 fluchten und die Stange vollständig aus dem Schaft gezogen werden kann. Das Federelement 16a und die Ausnehmung 15a (Figuren 2b und 3b) werden durch Verdrehen des Schaftes 3 relativ zu Stange 6 außer Eingriff gebracht, wobei das Herausgleiten des Federelementes 16a aus der Ausnehmung 15a erleichtert wird, wenn diese Teile im Bereich ihrer gegenseitigen Berührungsflächen mit schrägen Gleitflächen bzw. Fasen versehen sind.

Bei der beschriebenen Zange handelt es sich um eine monopolare Zange, bei der die Maulteile 5 eine Elektrode bilden, so daß eine weitere Elektrode am Körper des Patienten anzulegen ist.

Der Schaft 3 und die Handhabe 2 sind aus einem nicht leitenden Werkstoff, beispielsweise Kunststoff gefertigt und können mit metallischen Einlagen verstärkt sein. Die Stromleitung von dem HF-Anschluß 31 zu den Maulteilen 5 erfolgt über die Verbindungsteile 32 bis 34, den Schaft 3 und/oder die Stange 6.

Wie bereits erwähnt wurde, kann der aus dem Schaft 3 und der Stange 6 gebildete Zangeneinsatz mit dem Drehelement 28 relativ zur Handhabe 1 verstellt werden. Dabei kann es für einige Anwendungsfälle zweckmäßig sein, wenn der Zangeneinsatz stufenweise über vorgegebene Drehwinkelbereiche verdreht werden kann, wobei zu diesem Zweck das Drehelement 28 mit einem Rastmechanismus zusammenarbeitet. Dieser besteht gemäß Figur 7 aus einer Kugel 35, die mit einer Feder 36 in Richtung auf die eine Stirnfläche des Drehelementes 28 gedrückt wird und in dort vorgesehene Ausnehmungen 37 einrasten kann.

Die erfindungsgemäße Zange kann bei entsprechender Ausbildung der Maulteile mit Schneiden auch zum Auftrennen bzw. Durchtrennen von Gewebe dienen. Auch weitere Ausführungsvarianten, beispielsweise zum Halten von Gewebe, sind möglich.

## Patentansprüche

1. Chirurgische Zange, insbesondere zum Koagulieren von Gewebe mit HF-Strom, bei der das Zangenmaul (4) durch zwei an einem Träger (7) angeordnete Maulteile (5) gebildet ist, von denen wenigstens eines relativ zum anderen und zum Träger mittels einer Stange (6) zum Öffnen und Schließen des Zangenmaules (4) verschwenkbar ist, bei der die durch einen Schaft (3) verlaufende Stange (6) bei Betätigung einer Handhabe (1) axial im Schaft (3) verstellbar ist, dadurch gekennzeichnet, daß der Träger (7) und das distale Ende des Schaftes (3) lösbar miteinander verriegelt und gegen Axialbewegung relativ zueinander gesichert sind, daß die Stange (6) und der Schaft (3) im Bereich der Handhabe (1) lösbar und drehschlüssig verbunden sind und daß der proximale Endbereich der Stange (6) als Kupplungsteil (17,18) ausgebildet ist, das lösbar mit der Handhabe (1) in Verbindung steht.

2. Zange nach Anspruch 1, dadurch gekennzeichnet, daß der Träger (7) und der Schaft (3) mittels einer bajonettartigen Verbindung verriegelt sind, wobei radiale Vorsprünge (13) am Träger in eine Ausnehmung (20) des Schaftes greifen.

3. Zange nach einem der Ansprüche 1 und 2, dadurch gekennzeichnet, daß ein Federelement (16, 16a) im Zusammenwirken mit einer Ausnehmung (15, 15a) die drehschlüssige Verbindung zwischen der Stange (6) und dem Schaft (3) bildet.

4. Zange nach einem der Ansprüche 1 bis 3, dadurch gekennzeichnet, daß ein durch den Schaft (3) einerseits und die Stange (6) mit den an ihr befestigten Teilen (7,8,9,10) andererseits gebildeter Zangeneinsatz (2) mittels einer Gewindehülse (23) an der Handhabe (1) befestigt ist, indem die Gewindehülse mit einem Gewindestutzen (27) am feststehenden Teil (24) der Handhabe (1) verschraubt ist.

5. Zange nach einem der Ansprüche 1 bis 4, dadurch gekennzeichnet, daß der Schaft (3) und die Stange (6) einen relativ zur Handhabe (1) verdrehbaren Zangeneinsatz bilden.

6. Zange nach Anspruch 5, dadurch gekennzeichnet, daß der Zangeneinsatz (3, 6) mit einem an der Handhabe (1) befindlichen Drehelement (28) stufenweise über vorgegebene Drehwinkelbereiche verdrehbar ist und daß das Drehelement mit einem Rastmechanismus (35, 36, 37) zusammenarbeitet.

7. Zange nach einem der Ansprüche 1 und 6, nämlich monopolare Zange mit einer durch die Maulteile (5) gebildeten Elektrode und einem an der Handhabe (1) angeordneten HF-Anschluß (31), dadurch gekennzeichnet, daß der HF-Anschluß (31) und die Stange (6) mit den Maulteilen (5) in elektrischer Verbindung stehen.
